Europäisches Patentamt

**(19)** European Patent Office

Office européen des brevets

**(11)** Numéro de publication : **0 324 701 B1**

## **(12)** FASCICULE DE BREVET EUROPEEN

**(45)** Date de publication du fascicule du brevet :
**27.11.91 Bulletin 91/48**

**(51)** Int. Cl.$^5$ : **C07C 265/14, C07C 263/20**

**(21)** Numéro de dépôt : **89420012.0**

**(22)** Date de dépôt : **12.01.89**

**(54)** Procédé de séparation du toluène diisocyanate contenu dans les résidus de sa fabrication.

**(30)** Priorité : **13.01.88 FR 8800466**

**(43)** Date de publication de la demande :
**19.07.89 Bulletin 89/29**

**(45)** Mention de la délivrance du brevet :
**27.11.91 Bulletin 91/48**

**(84)** Etats contractants désignés :
**BE DE ES FR IT**

**(56)** Documents cités :
**EP-A- 0 000 463**
**FR-A- 1 555 515**
**FR-A- 2 091 813**
**FR-A- 2 149 148**

**(73)** Titulaire : **RHONE-POULENC CHIMIE**
**25, quai Paul Doumer**
**F-92408 Courbevoie Cédex (FR)**

**(72)** Inventeur : **Collas, Gérard**
**406, avenue du 8 Mai**
**F-69300 Caluire (FR)**
Inventeur : **Gros, Georges**
**124, avenue Général Leclerc**
**F-92340 Bourg la Reine (FR)**
Inventeur : **Sagi, Ferenc**
**133, avenue Ferdinand Buisson**
**F-69003 Lyon (FR)**

**(74)** Mandataire : **Varnière-Grange, Monique et al**
**RHONE-POULENC INTERSERVICES Service**
**Brevets Chimie Centre de Recherches des**
**Carrières B.P. 62**
**F-69192 Saint-Fons Cédex (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de séparation du toluène diisocyanate (TDI) des résidus de sa fabrication, en vue de sa récupération.

Il est bien connu que lors de la fabrication du TDI il se forme des quantités notables de composés lourds. Ces quantités, variables selon la nature précise du procédé de fabrication mis en oeuvre représentent généralement de 10 à 15% en poids voire d'avantage. Ces composés lourds sont habituellement concentrés par distillation du TDI jusqu'à la concentration type de 30 à 40% de TDI et de 60 à 70% de composés lourds.

Il est difficile de poursuivre la distillation pour obtenir des mélanges plus fortement concentrés en composés lourds, en raison de l'augmentation de la viscosité du mélange. Il faut donc mettre en oeuvre d'autres techniques pour récupérer le TDI contenu dans un tel mélange, pour disposer d'un résidu combustible renfermant les composés lourds en cause et ne contenant plus de TDI et pour éviter à la fois la pollution par des vapeurs toxiques et les pertes de produit onéreuses.

Diverses techniques ont été antérieurement proposées à cette fin mais chacune d'entre elles présente au moins l'un des inconvénients ci-après :
— elle est discontinue,
— elle est coûteuse en matière première et/ou en énergie,
— elle est très difficile à mettre en oeuvre à l'échelle industrielle.

Une analyse de ces diverses solutions et des inconvénients spécifiques qu'elles présentent est développée en préambule du brevet européen n°0000463 qui propose également une solution distincte à ce problème à savoir :

le traitement en continu de résidus de cette fabrication dans un évaporateur agité et raclé, à une température débutant à 100°-130°C, sous une pression de 666,5 à 2666 Pa, température, portée ensuite progressivement jusqu'à une température de 220° à 260°C avec un temps de de séjour minimum de 15 mn dans l'évaporateur, pour évaporer le TDI tout en évacuant en continu les résidus, notamment par le recours à un système d'extrusion.

Comme cela apparaît à la lecture même du brevet européen en cause, ce procédé qui permet d'atteindre de bons résultats à l'échelle industrielle présente néanmoins les inconvénients suivants :
— Son fonctionnement satisfaisant impose des contrôles stricts à la fois des conditions physico-chimiques (pression, température, temps de séjour) et des conditions mécaniques (agitation, transport des produits de viscosité croissante dans l'évaporateur, raclage).

En effet, le moindre écart dans ces conditions est susceptible de causer soit une polymérisation pouvant aller jusqu'à une prise en masse du résidu dans l'appareil, soit une décomposition du TDI en gaz générateurs de mousse dans le mélange résiduaire visqueux.
— Le déplacement du résidu visqueux le long de la paroi de l'évaporateur doit être contrôlé de telle sorte qu'au fur et à mesure que l'évaporation du TDI se produit il soit possible d'augmenter la température pour finir ladite évaporation sans courir le risque d'une décomposition.

Par ailleurs, ce procédé se révèle à l'usage présenter l'inconvénient supplémentaire suivant. Son fonctionnement n'est possible que pour certaines catégories de résidus à savoir ceux qui offrent un profil de viscosité croissante au fur et à mesure que l'évaporation du TDI progresse. Pour des résidus de fabrication industrielle ne présentant pas un tel profil le procédé en question ne peut être mis en oeuvre.

C'est pourquoi, un besoin se fait sentir de pouvoir disposer d'un procédé de séparation du TDI contenu dans les résidus de sa fabrication susceptible d'être mis en oeuvre en continu, indépendamment de la nature ou de la provenance précise des résidus à traiter, qui soit à la fois efficace, simple à mettre en oeuvre à l'échelle industrielle et peu onéreux et qui, le cas échéant donne naissance à un rejet final sous une forme aisément manipulable.

La présente invention a donc pour objet un procédé de séparation du toluènediisocyanate contenu dans les résidus de sa fabrication caractérisé en ce que lesdits résidus sont traités par un gaz inerte à l'état liquide ou supercritique.

Les résidus de la fabrication du TDI peuvent en pratique présenter divers aspects ou compositions. En effet, lorsqu'on produit du TDI (mélange des isomères – 2,4 et – 2,6 du diisocyanatoluène) par phosgénation de la toluène diamine (mélange des isomères – 2,4 et – 2,6 à raison de 80 parties du premier pour 20 parties du second, environ) par exemple dans de l'ortho-dichlorobenzène comme diluant de la réaction, le mélange réactionnel est soumis à au moins une distillation pour en éliminer l'acide chlorhydrique, le phosgène résiduel et au moins une partie du diluant de la réaction. Il subsiste alors un mélange constitué par de 8 à 20% (pds) de TDI, de 0,5 à 3% de produits lourds, le reste à 100% étant de l'ortho-dichlorobenzène.

Ce mélange peut être concentré par élimination de l'ortho-dichlorobenzène qu'il contient et ne renfermer alors que des produits lourds et du TDI. Le résidu débarrassé de l'ortho-dichlorobenzène peut être à son tour

2

concentré comme indiqué en tête du présent mémoire par distillation du TDI, la concentration type pouvant atteindre de 50 à 70% de composés lourds pour de 30 à 50% de TDI, ce résidu à la fois concentré et débarrassé de l'orthodichlorobenzène se présentant sous forme de goudrons dont le point de ramollissement se situe entre (environ) 200 et 250°C.

Bien que ces différents types de résidus puissent être traités selon le procédé, objet de la présente invention, ledit procédé revêt un intérêt plus prononcé pour traiter des mélanges résiduaires dont la composition est la suivante :

— de 5 à 100% poids d'un mélange

de 95 à 50% poids de TDI et

de 5 à 50% poids de produits lourds,

pour

— de 0 à 95% poids d'un diluant.

Bien entendu lorsqu'un diluant est présent dans le mélange résiduaire à traiter il est avantageusement le diluant de la réaction de phosgénation. Généralement le diluant utilisé est un composé hydrocarboné aromatique ou aromatique chloré tel que le toluène, le monochlorobenzène et l'orthodichlorobenzène. On peut néanmoins recourir à d'autres diluants notamment à des esters tels le phtalate d'éthyle, d'isopropyle, de butyle ou d'isobutyle ou l'isophtalate correspondant, l'adipate de butyle ou d'isobutyle, le butyrate ou l'isobutyrate de méthyle, d'éthyle, de butyle ou d'isobutyle, l'éthyl-2 hexanoate d'éthyl-2 hexyle, ou à d'autres hydrocarbures chlorés tel le chlorure de méthylène et, en particulier, le trichloro-1, 2,4 benzène.

Lorsqu'un diluant est présent dans le mélange résiduaire à traiter ce qui constitue un mode de mise en oeuvre particulièrement avantageux du présent procédé il représente, de préférence de 5 à 30% (poids) de la composition.

Il a donc maintenant été trouvé que l'opération de séparation du TDI contenu dans les résidus de sa fabrication est facile à réaliser par traitement du résidu par un gaz inerte à l'état liquide ou supercritique. Comme gaz inertes on peut citer à titre d'exemple le gaz carbonique, le butane, l'éthane, le propane, l'éthylène, le protoxyde d'azote, $SF_6$, et les "fréons". Le gaz carbonique qui est bon marché, non toxique et ininflammable est le composé dont l'usage est préconisé. On a constaté que, le $CO_2$ supercritique dissout et extrait convenablement le TDI présent dans le "condensat".

Il a également été trouvé que l'opération de séparation en cause et, en particulier, l'extraction par le gaz carbonique à l'état liquide ou supercritique est avantageusement conduite en présence d'un co-extractant choisi parmi les diluants de la réaction de phosgénation, le co-extractant pouvant être de nature distincte du diluant utilisé pour conduire ladite réaction.

Le co-extractant représente, en général, de 5 à 70% (pds) du gaz carbonique à l'état liquide ou supercritique et, de préférence, au moins 10% (pds) dudit gaz. On n'observe pas d'avantage particulier lorsque cette teneur dépasse 30% (pds) du gaz carbonique à l'état liquide ou supercritique.

Le co-extractant est avantageusement choisi parmi le toluène, l'orthodichlorobenzène, le trichloro-1,2,4 benzène et le monochlorobenzène.

Le co-extractant est avantageusement de même nature que le diluant utilisé lors de la fabrication du TDI par phosgénation de la diamine correspondante, comme indiqué en tête du présente mémoire. Pour une bonne mise en oeuvre du procédé selon l'invention, la quantité de co-extractant sera en pratique d'autant plus élevée que la proportion de diluant dans le résidu à traiter est faible.

Il a en effet été constaté par la Demanderesse qu'en présence d'un tel co-extractant, le rejet final contient des quantités notablement inférieures de TDI à celle contenues par le rejet produit en l'absence d'un tel co-extractant.

En outre, en présence d'un tel co-extractant les risques de gélification du mélange dans la colonne d'extraction sont considérablement limités.

Le procédé selon la présente invention comporte donc un traitement de résidus définis ci-avant par un gaz inerte à l'état liquide ou supercritique qui est avantageusement le gaz carbonique. Le cas échéant, cette extraction est conduite en présence d'un co-extractant au sens indiqué précédemment ; les conditions exposées pour $CO_2$ seul s'appliquant également à ce cas.

Cette extraction peut se faire en continu ou en discontinu.

A l'état liquide, le $CO_2$ est mis en oeuvre à une température comprise en 0 et 31°C, et sous une pression de 30 à 500 bars. Il est souvent préférable d'opérer entre 20 et 31°C lorsque la viscosité du résidu est importante. La pression peut aller de 60 à 300 bars.

Cette extraction peut se faire à l'état surpercritique à une température supérieure à 31,4°C (température critique du $CO_2$) et à une pression comprise entre 73 et 500 bars. De préférence, la température est comprise entre 31,4°C et 100°C et la pression entre 73 et 350 bars

Le résidu renfermant le TDI est traité par le $CO_2$ liquide ou à l'état supercritique ; on peut opérer en discontinu, c'est-à-dire en mélangeant dans un réacteur le résidu avec le $CO_2$ à l'état liquide ou supercritique.

Généralement, dans le cadre du procédé selon l'invention on préfère mettre en oeuvre le gaz d'extraction à l'état supercritique.

L'extraction peut être conduite en continu de manière conventionnelle, dans un appareillage connu en soi.

Le gaz d'extraction à l'état liquide ou supercritique est envoyé dans l'appareil d'extraction qui peut être par exemple une colonne remplie d'un garnissage permettant un meilleur contact entre le résidu et le gaz d'extraction. Le résidu peut être introduit à l'autre extrémité de la colonne : on a alors une extraction à contre-courant. Moins fréquemment, on peut l'introduire à la même extrémité que le gaz d'extraction : on a alors une extraction à co-courant. Le rejet est récupéré à une extrémité de la colonne d'extraction, tandis que le gaz d'extraction chargé du TDI, et éventuellement de co-extractant est traité pour le séparer des produits extraits.

On peut pour cela agir soit par diminution de sa pression soit par augmentation de sa température, soit à la fois par diminution de sa pression et augmentation de sa température. Ces conditions ont pour but de modifier le pouvoir solvant du gaz d'extraction.

La diminution de pression ou détente, peut se faire en une ou plusieurs étapes et le gaz d'extraction peut être détendu jusqu'à une pression égale à la pression atmosphérique ou jusqu'à une pression plus élevée, sous laquelle il sera recyclé dans le cas d'un procédé continu.

En effet, si le gaz d'extraction est recyclé, il est économiquement préférable de ne pas le détendre jusqu'à la pression atmosphérique, ce qui nécessiterait une plus grande dépense d'énergie pour le recomprimer dans le cycle suivant le procédé. Il est préférable de le détendre jusqu'à une pression sous laquelle les composés extraits ne sont pas, ou sont très peu solubles.

Le rejet liquéfié par le gaz d'extraction et le co-extractant dans la colonne est susceptible de se gélifier ou de solidifier lorsque la détente est opérée sans précautions particulières. Toutefois, il est possible de réaliser la détente de manière contrôlée, voire dans un liquide véhiculaire-et d'obtenir le rejet en cause sous forme d'une solution ou d'une dispersion manipulable.

Divers modes d'exécution apparaîtront à la lecture du présent mémoire parmi lesquels on citera, à titre de variante du présent procédé, le mode de réalisation selon lequel la colonne est initialement remplie au moins partiellement du résidu à traiter à travers lequel on fera buller le $CO_2$, le cas échéant, avec le co-extractant à contre courant de l'alimentation en résidu et, à titre de variante avantageuse du présent procédé, le mode de réalisation selon lequel la colonne est initialement remplie du moins partiellement de $CO_2$, le cas échéant, en mélange avec un co-extractant, l'alimentation du résidu, le cas échéant dilué dans le co-extractant, étant réalisée en tête de la colonne.

Le procédé qui vient d'être décrit permet de traiter, notamment, des résidus obtenus avant la distillation du TDI pour les concentrer ; il permet également par le choix d'un co-extractant approprié tel l'orthodichlorobenzène de mener à bien le traitement et de recycler ledit co-extractant à la fabrication du TDI ce qui présente un avantage certain.

Les exemples ci-après illustrent la présente invention.

## EXEMPLE 1 :

Dans une colonne en acier inoxydable de 16 mm de diamètre et de 70 mm de hauteur remplie de garnissage d'anneaux Raschig en verre (6 mm × 6 mm) on introduit 250 g de goudrons de composition suivante :
— orthodichlorobenzène 9% (pds)
— TDI 55% (pds)
— composés lourds 36% (pds)

La colonne est thermostatée à la température de 50°C, puis on admet du $CO_2$ à l'état supercritique jusqu'à la pression de 190 bars. Cette pression sera pendant toute la durée de l'essai, régulée par une vanne de détente à la pression de 190 bars.

L'ensemble du système étant en équilibre on introduit simultanément en bas de la colonne le gaz carbonique à l'état supercritique avec un débit de 1100 g/h environ et l'orthodichlorobenzène (ODCB) avec un débit de 350 g/h.

Les extraits sont récupérés, au delà de la vanne de détente, dans des poudriers successifs à la pression atmosphétique.

Lorsque le rapport (Poids de $CO_2$ introduit)/charge) est égal à 50, la composition du résidu dans la colonne est la suivante :

```
- orthodichlorobenzène = 33    %  (pds)
- TDI                   =  0,8 %  (pds)
- lourds                = 66,2 %  (pds).
```

A ce stade, l'essai est poursuivi en introduisant en continu en tête de colonne, les goudrons avec un débit de 23 g/h.

A la base de la colonne, on alimente ensemble le $CO_2$ et l'ODCB avec les débits respectifs de 1100 g/h et 330 g/h, soit un rapport ODCB/$CO_2$ de 30/100, qui passent dans le produit à traiter. Au delà du déverseur, régulateur de pression, l'extrait récupéré à la pression atmosphérique avec un débit de 320 g/h environ contient 4% de TDI environ et 96% d'ODCB.

Le résidu de l'extraction récupéré en pied de colonne à une teneur en TDI de 1,2% (pds), le reste étant de l'ODCB (33% pds) et des lourds (65,8% pds).

## EXEMPLE 2 :

En utilisant la colonne d'extraction décrite dans l'exemple 1 avec les mêmes conditions de pression et de température, on introduit en continu en pied de colonne un mélange de $CO_2$ et d'ODCB avec les débits respectifs de 1250 g/h et 350 g/h, soit un rapport ODCB/$CO_2$ de 28/100.

A contre courant, en tête de colonne on alimente en continu les goudrons de composition identique à ceux de l'exemple 1 — débit = 100 g/h — le rapport débit $CO_2$/débit d'alimentation est donc de 12,5.

Dans ces conditions, on récupère en continu, en pied de colonne, un résidu d'extraction dont la composition en poids est la suivante :

```
- ODCB      48   %
- TDI       2,3  %
- lourds    50,2 %
```

## EXEMPLE 3 :

En utilisant la colonne d'extraction décrite dans l'exemple 1 avec les conditions de pression et de température similaires (200 bars ; 50°C) on introduit simultanément en pied de colonne un mélange de $CO_2$ et d'ODCB avec les débits respectifs de 1125 g/h et de 180 g/h soit un rapport ODCB/$CO_2$ de 16/100.

A contre courant, en tête de colonne, on alimente en continu des goudrons de TDI dont la composition est la suivante :

— 50% (pds) de TDI

— 50% de goudrons.

Le débit est fixé à 21,5 g/h. Après équilibrage de la marche de la colonne, un échantillon du résidu contient 1,7% de TDI résiduel.

## Revendications

1. Procédé de séparation du toluène diisocyanate contenu dans les résidus de sa fabrication caractérisé en ce que lesdits résidus sont traités par un gaz inerte à l'état liquide ou supercritique.

2. Procédé selon la revendication 1, caractérisé en ce que les résidus ont pour composition :

— de 5 à 100% poids d'un mélange

de 95 à 50% poids de TDI et

de 5 à 50% poids de produits lourds,

pour

— de 0 à 95% poids d'un diluant.

3. Procédé selon la revendication 2, caractérisé en ce que le diluant est choisi parmi le toluène, l'orthodichlorobenzène, le trichloro-1,2,4 benzène et le monochlorobenzène.

4. Procédé selon la revendication 2 ou 3, caractérisé en ce que le diluant est l'orthodichlorobenzène.

5. Procédé selon l'une quelconque des revendications 2 à 4, caractérisé en ce que le diluant représente

5

de 5 à 30% en poids de la composition.

6. Procédé selon l'une quelconque des revendications précédentes, caractérisé en ce que le gaz inerte utilisé à l'état liquide ou supercritique est le gaz carbonique.

7. Procédé selon la revendication 6, caractérisé en ce que l'extraction par le gaz carbonique à l'état liquide ou supercritique est conduite en présence d'un co-extractant, choisi parmi les diluants de la réaction de phosgénation, le coextractant pouvant être de nature distincte du diluant utilisé pour conduire la dite réaction.

8. Procédé selon la revendication 7, caractérisé en ce que le co-extractant est choisi parmi le toluène, l'orthodichlorobenzène, le trichloro-1,2,4 benzène et le monochlorobenzène.

9. Procédé selon les revendication 7 ou 8 caractérisé en ce que le co-extractant représente de 5 à 70% en poids du gaz carbonique à l'état liquide ou supercritique.

10. Procédé selon la revendication 9, caractérisé en ce que le co-extractant représente au moins 10% en poids du gaz carbonique à l'état liquide ou supercritique.

11. Procédé selon l'une quelconque des revendications 7 à 10, caractérisé en ce que le co-extractant est l'orthodichlorobenzène.

12. Procédé selon l'une quelconque des revendications 7 à 11, caractérisé en ce que le co-extractant est de même nature que le diluant utilisé lors de la phosgénation.

13. Procédé selon l'une quelconque des revendications 7 à 12, caractérisé en ce que le gaz carbonique utilisé est à l'état supercritique.

14. Procédé selon l'une quelconque des revendications 6 à 12 caractérisé en ce que l'on opère à une température comprise entre 0 et 31°C et sous une pression de 30 à 500 bars.

15. Procédé selon la revendication 14, caractérisé en ce que l'on opère à une température comprise entre 20 et 31°C et sous une pression de 60 à 300 bars.

16. Procédé selon l'une quelconque des revendications 6 à 13, caractérisé en ce que l'on opère à une température supérieure à la température critique du gaz carbonique de préférence inférieure à 100°C et sous une pression de 73 à 500 bars et de préférence de 73 à 350 bars.


## Patentansprüche

1. Verfahren zur Abtrennung von Toluoldiisocyanat, das in den Rückständen seiner Herstellung enthalten ist, dadurch gekennzeichnet, daß diese Rückstände mit einem Inertgas im flüssigen oder überkritischen Zustand behandelt werden.

2. Verfahren nach Anspruch 1, dadurch gekennzeichnet, daß die Rückstände folgende Zusammensetzung aufweisen :

— 5 bis 100 Gew.-% eines Gemisches aus
95 bis 50 Gew.-% TDI und
5 bis 50 Gew.-% schwere Produkte,
auf
— 0 bis 95 Gew.-% eines Verdünnungsmittels.

3. Verfahren nach Anspruch 2, dadurch gekennzeichnet, daß das Verdünnungsmittel unter Toluol, Orthodichlorbenzol, 1,2,4-Trichlorbenzol und Monochlorbenzol ausgewählt wird.

4. Verfahren nach Anspruch 2 oder 3, dadurch gekennzeichnet, daß das Verdünnungsmittel Orthodichlorbenzol ist.

5. Verfahren nach einem der Ansprüche 2 bis 4, dadurch gekennzeichnet, daß das Verdünnungsmittel 5 bis 30 Gew.-% der Zusammensetzung ausmacht.

6. Verfahren nach einem der vorangehenden Ansprüche, dadurch gekennzeichnet, daß das im flüssigen oder überkritischen Zustand eingesetzte Inertgas Kohlendioxid ist.

7. Verfahren nach Anspruch 6, dadurch gekennzeichnet, daß die Extraktion mittels Kohlendioxid im flüssigen oder überkritischen Zustand in Gegenwart eines Co-Extraktionsmittels ausgeführt wird, das unter den Verdünnungsmitteln der Phosgenierungsreaktion ausgewählt wird, wobei das Co-Extraktionsmittel von dem für die Durchführung dieser Reaktion verwendeten Verdünnungsmittel verschieden sein kann.

8. Verfahren nach Anspruch 7, dadurch gekennzeichnet, daß das Co-Extraktionsmittel unter Toluol, Orthodichlorbenzol, 1,2,4-Trichlorbenzol und Monochlorbenzol ausgewählt wird.

9. Verfahren nach den Ansprüchen 7 oder 8, dadurch gekennzeichnet, daß das Co-Extraktionsmittel 5 bis 70 Gew.-% des Kohlendioxids im flüssigen oder überkritischen Zustand ausmacht.

10. Verfahren nach Anspruch 9, dadurch gekennzeichnet, daß das Co-Extraktionsmittel mindestens 10 Gew.-% des Kohlendioxids im flüssigen oder überkritischen Zustand ausmacht.

11. Verfahren nach einem der Ansprüche 7 bis 10, dadurch gekennzeichnet, daß das Co-Extraktionsmittel Orthodichlorbenzol ist.

12. Verfahren nach einem der Ansprüche 7 bis 11, dadurch gekennzeichnet, daß das Co-Extraktionsmittel von gleicher Beschaffenheit wie das bei der Phosgenierung verwendete Verdünnungsmittel ist.

13. Verfahren nach einem der Ansprüche 7 bis 12, dadurch gekennzeichnet, daß das eingesetzte Kohlendioxid sich im überkritischen Zustand befindet.

14. Verfahren nach einem der Ansprüche 6 bis 12, dadurch gekennzeichnet, daß man bei einer Temperatur im Bereich von 0 bis 31°C und unter einem Druck von 30 bis 500 bar arbeitet.

15. Verfahren nach Anspruch 14, dadurch gekennzeichnet, daß man bei einer Temperatur im Bereich von 20 bis 31°C und unter einem Druck von 60 bis 300 bar arbeitet.

16. Verfahren nach einem der Ansprüche 6 bis 13, dadurch gekennzeichnet, daß man bei einer Temperatur oberhalb der kritischen Temperatur des Kohlendioxids, vorzugsweise unterhalb 100°C, und unter einem Druck von 73 bis 500 bar, vorzugsweise von 73 bis 350 bar, arbeitet.

## Claims

1. Process for the separation of toluene diisocyanate present in the residues for its manufacture, characterised in that the said residues are treated with an inert gas in the liquid or supercritical state.

2. Process according to Claim 1, characterised in that the residues have the composition :
— from 5 to 100% by weight of a mixture
of 95 to 50% by weight of TDI and
of 5 to 50% by weight of heavy products,
for
— from 0 to 95% by weight of a diluent.

3. Process according to Claim 2, characterised in that the diluent is chosen from toluene, orthodichlorobenzene, 1,2,4-trichlorobenzene and monochlorobenzene.

4. Process according to Claim 2 or 3, characterised in that the diluent is ortho-dichlorobenzene.

5. Process according to any one of Claims 2 to 4, characterised in that the diluent represents from 5 to 30% by weight of the composition.

6. Process according to any one of the preceding claims, characterised in that the inert gas employed in the liquid or supercritical state is carbon dioxide.

7. Process according to Claim 6, characterised in that the extraction with carbon dioxide in the liquid or supercritical state is performed in the presence of a coextractant chosen from diluents for the phosgenation reaction, it being possible for the coextractant to be different in kind from the diluent employed for performing the said reaction.

8. Process according to Claim 7, characterised in that the coextractant is chosen from toluene, orthodichlorobenzene, 1,2,4-trichlorobenzene and monochlorobenzene.

9. Process according to Claim 7 or 8, characterised in that the coextractant represents from 5 to 70% by weight of the carbon dioxide in the liquid or supercritical state.

10. Process according to Claim 9, characterised in that the coextractant represents at least 10% by weight of the carbon dioxide in the liquid or supercritical state.

11. Process according to any one of Claims 7 to 10, characterised in that the coextractant is orthodichlorobenzene.

12. Process according to any one of Claims 7 to 11, characterised in that the coextractant is of the same kind as the diluent employed during the phosgenation.

13. Process according to any one of Claims 7 to 12, characterised in that the carbon dioxide employed is in the supercritical state.

14. Process according to any one of Claims 6 to 12, characterised in that the operation is carried out at a temperature of between 0 and 31°C and at a pressure of 30 to 500 bars.

15. Process according to Claim 14, characterised in that the operation is carried out at a temperature of between 20 and 31°C and at a pressure of 60 to 300 bars.

16. Process according to any one of Claims 6 to 13, characterised in that the operation is carried out at a temperature above the critical temperature of carbon dioxide, preferably below 100°C and at a pressure of 73 to 500 bars and preferably of 73 to 350 bars.